# EUROPEAN PATENT SPECIFICATION

(11) **EP 1 809 328 B1**
(45) Date of publication and mention of the grant of the patent: **13.03.2013**
(21) Application number: 05734832.8
(22) Date of filing: 14.04.2005
(51) Int. Cl.: A23L 1/0526, A61K 31/7004, A61K 31/702, A61K 31/715

(54) **MANNOOLIGOSACCHARIDE COMPOSITION FOR BODY FAT REDUCTION**
ZUSAMMENSETZUNG ENTHALTEND MANNOOLIGOSACCHARIDE ZUR KÖRPERFETTREDUZIERUNG
COMPOSITION COMPRENANT UN MANNOOLIGOSACCHARIDE POUR REDUIRE LA GRAISSE CORPORELLE

(30) Priority: 17.09.2004 JP 2004271412
(43) Date of publication of application: 25.07.2007
(73) Proprietor: Ajinomoto General Foods, Inc., Tokyo (JP); Kraft Foods Global Brands LLC, Northfield, IL 60093 (US)
(72) Inventor: ASANO, Ichiro, (JP); FUJII, Shigeyoshi, (JP); MUTOH, Katsuhito, 1-10-14 Shimura, Itabashi-ku Tokyo (JP); TAKAO, Izumi, Suzuka-shi, Mie (JP); OZAKI, Kazuto, Yatomi-cho Ama-gun, Aichi (JP); NAKAMURO, Kenichi, Edogawa-ku, Tokyo (JP); MATSUSHIMA, Toshiyuki, Nakano-ku Tokyo (JP)
(74) Representative: Murray, Adrian D'Coligny
(86) International application number: PCT/US2005/012823
(87) International publication number: WO 2006/036208

(56) References cited:
- JP-A- 2002 262 828
- US-A1- 2003 073 643
- US-A1- 2004 081 994
- US-B2- 6 746 696
- SACHSLEHNER A ET AL: "Hydrolysis of isolated coffee mannan and coffee extract by mannanases of Sclerotium rolfsii" JOURNAL OF BIOTECHNOLOGY, ELSEVIER SCIENCE PUBLISHERS, AMSTERDAM, NL, vol. 80, no. 2, 23 June 2000 (2000-06-23), pages 127-134, XP004213502 ISSN: 0168-1656
- DATABASE WPI Week 200313 Derwent Publications Ltd., London, GB; AN 2003-132119 XP002451442 -& JP 2002 262827 A (AJINOMOTO GENERAL FOODS INC) 17 September 2002 (2002-09-17)
- TAKAHASHI H ET AL: "Protein and energy utilization of growing rats fed on the diets containing intact or partially hydrolyzed guar gum" COMPARATIVE BIOCHEMISTRY AND PHYSIOLOGY, vol. 107A, no. 1, 1994, pages 255-260, XP002451439
- DATABASE CA [Online] CHEMICAL ABSTRACTS SERVICE, COLUMBUS, OHIO, US; 1993, YAMATOYA ET AL: "Effects of partially hydrolyzed guar gum on postprandial plasma glucose and lipid levels in humans" XP002451440 retrieved from STN Database accession no. 119:116199 & NIPPON EIYO, SHOKUYO GAKKAISHI, vol. 46, no. 3, 1993, pages 199-203,

## Description

The present invention relates to the use of mannooligosaccharide compositions and foods and beverages containing mannooligosaccharides for reducing body fat and especially abdominal fat. The mannooligosaccharides may be produced by hydrolysis of mannan materials and incorporated into foods and beverages.

### BACKGROUND

In recent years, westernized diets, lack of exercise and greater stress have resulted in increased weight gains and obesity. Excess weight and obesity are risk factors for diseases such as hyperglycemia, hypertension and diabetes. Dietary remedies have been provided to limit weight gain and treat obesity. Many of these dietary remedies are natural products or are derived from natural products.

The coffee industry is one example of an industry that processes and produces large amounts of natural products. Makers of soluble coffee and canned coffee discharge large amounts of spent coffee grounds. These residues are mostly incinerated or disposed as industrial waste. Coffee beans and resulting coffee residues contain water insoluble β-mannan. The mannooligosaccharide (MOS) from hydrolyzed coffee mannan are indigestible oligosaccharides. The present invention utilizes mannan to provide compositions having fat reducing effects.

### SUMMARY

According to the present invention there is provided a composition comprising a monosaccharide having a degree of polymerisation of 2 to 10 monosaccharides, wherein at least 60 weight percent of the monosaccharides are mannose, for use in reducing body fat ratios or abdominal fat in a human.

The mannooligosaccharide composition may be added to food compositions, especially beverages, and consumed orally. Administration of the mannooligosaccharides is effective for reducing overall body fat, as expressed by body fat ratios, by at least 4%, preferably 4% to 8%, abdominal fat by at least 10%, preferably 10% to 15%, and weight loss.
Preferably at least 70 weight percent of the monosaccharides are mannose, and most preferably at least 80 weight percent of the monosaccharides are mannose. The mannooligosaccharide may also include one or more monosaccharides such as glucose, galactose, fructose and mixtures thereof. The mannooligosaccharide composition may be incorporated into foods, beverages, feeds, medicines and cosmetics. The mannooligosaccharide compositon may be provided as a hydrolysate which is formed by hydrolysis of a mannan material. The mannan material may be provided from sources that include raw coffee beans, roasted coffee beans, spent coffee grounds, copra meal, copra flakes, coconut, Huacra palm, yarns, konjak, lily, narcissus, licorice, locust bean gum, guar gum, and mixtures thereof. In an important aspect, the source of mannan material is provided from coffee materials and especially from spent coffee grounds. Mannan composition derived from guar gum and effective as body for reducing agents are described in Takahashi, H. et al., Comp. Biochem. Physiol. Vol. 107A, No1, pp. 255-260, 1994.

The hydrolysate containing mannooligosaccharides may be produced using a hydrolysis methods such as acid hydrolysis, thermal hydrolysis, enzyme hydrolysis, microbial fermentation hydrolysis, and combinations of such methods. Thermal hydrolysis may be conducted at a temperature of 200°C to 220°C, preferably 210°C. Hydrolysates may be decolorized, deodorized and/or concentrated by contacting the hydrolysate with activated carbon, adsorptive resins, ion-exchange resin, ion-exchange membranes, and combinations thereof.

Where the mannooligosaccharide is provided in a food composition, the food composition may include at least 1 gram of mannooligosaccharide per 100 grams of food composition. The mannooligosaccharides may be incorporated into a variety of solid foodstuffs and/or beverages. In an important aspect, the mannooligosaccharides are incorporated into coffee.

The mannooligosaccharide composition may be produced by a method which includes hydrolyzing a mannan material in an amount effective for forming a hydrolysate containing mannooligosaccharides where each mannooligosaccharide has a degree of polymerisation of 2 to 10 monosaccharides with at least 60 weight percent of the monosaccharides being mannose, preferably 70 weight percent of the monosaccharides being mannose, and most preferably 80 weight percent of the monosaccharides being mannose. The mannan material may be provided from raw coffee beans, roasted coffee beans, spent coffee grounds, copra meal, copra flakes, coconut, Huacra palm, yarns, konjak, lily, narcissus, licorice, locust bean gum, guar gum, and mixtures thereof. A preferred source of mannan material is spent coffee grounds. The hydrolysate containing mannooligosaccharides is produced using a hydrolysis methods such as acid hydrolysis, thermal hydrolysis, enzyme hydrolysis, microbial fermentation hydrolysis, and mixtures thereof. A preferred hydrolysis method includes thermal hydrolysis. The resulting hydrolysate may be deodorized, decolorized and/or concentrated using activated carbon, adsorptive resins, ion-exchange resin, ion-exchange membranes, and combinations thereof. The resulting hydrolysate may be used as an aqueous composition or may be dried or lyophilized.

The mannooligosaccharide may be orally administered at a rate of 1 to 10 grams per day, preferably 2 to 8 grams per day, and more preferable 3 to 6 grams per day, as part of a food composition, such as for example a beverage such as coffee.

### BRIEF DESCRIPTION OF FIGURES

Figure 1 illustrates changes of relative abdominal total fat area calculated from CT scan photos in a control group and MOS group.

Figure 2 illustrates changes of relative abdominal subcutaneous fat area of a control group and MOS group.

Figure 3 illustrates changes of relative abdominal visceral fat area of a control group and a MOS group.

Figure 4 shows the effect of MOS on pancreatic lipase.

Figure 5 illustrates the effect of MOS on human plasma triglycerol levels after oral administration of a high fat diet.

### DETAILED DESCRIPTION

Compositions are provided, the use of which provide fat reducing effects and which may be considered as having physiological functionality in reducing obesity. The compositions are oligosaccharides that mainly include mannose units. These mannose-based oligosaccharides or mannooligosaccharides are non-carcinogenic, and low calorie; they are selectively utilized by intestinal micoflora, suppress serum lipid levels, and promote mineral adsorption.

### Definitions

Mannooligosaccharides are provided which are oligosaccharides. As used herein, "oligosaccharides" refers to polymers of monosaccharides that have a degree of polymerization (DP) of 2 to 10, more preferably a DP of 2 to 9, and more preferably a DP of 2 to 6.

The mannooligosaccharides will often include a plurality of oligosaccharides with different degrees of polymerization.

### Mannan Material

As used herein, "mannan" refers to polysaccharides that include mannose monosaccharide residues. The mannose residues may be in the form of D-mannose, galactomannan, glucomannan, and mixtures thereof. The polysaccharides that include mannose may be entirely formed of mannose monosaccharide residues or may be a combination of mannose monosaccharide residues and other monosaccharides, such as for example, galactose, glucose and fructose.

Mannooligosaccharides may be produced by the hydrolysis of mannan. Sources of mannan include raw coffee beans, roasted coffee beans, spent coffee grounds, copra meal, copra flakes, coconut, Huacra palm, yarns, konjak, lily, narcissus, licorice, locust bean gum, guar gum, and mixtures thereof. Copra meal and copra flakes may be obtained from coconut. Huacra Palm of South African Arecacease (Palmae) plant, yam mannan, and Chinese yam mannan all provide a source of mannan material.

In an important aspect, roasted and ground coffee beans and spent coffee grounds which remain after brewing coffee are a preferred source of mannan. Any types of coffee beans from any source may be utilized. Examples of coffee that can be used include Arabica, Robusta, Liveria and the like. A single type of coffee or blends of different types of coffee may be utilized. Coffee beans of inferior quality, or size, including those having little or no commercial value, may also be used. In another important aspect, mannan may be provided from coffee extraction residues obtained after extraction treatment of roasted and ground coffee in conventional processes for producing liquid coffee or instant coffee.

### Hydrolysis of Mannan Material

Hydrolysis of the mannan material may be conducted using hydrolysis methods that may include acid hydrolysis, thermal hydrolysis, enzyme hydrolysis, microbial fermentation hydrolysis, and mixtures thereof. Hydrolysis methods using acid and/or high temperatures are described in Japanese Patent Application Nos. Sho 61-96947 and Hei 02-200147. Further, acid hydrolysis methods are described in U.S. Patent No. 4,508,745 and high temperature hydrolysis methods are described in EP0363529.

Spent coffee grounds may be hydrolyzed in a reaction vessel with or without acid and with or without pressure. Hydrolysis may also be carried out in any other reaction vessel suitable for such processing, such as for example, a plug flow reactor. Hydrolysis is effective for hydrolyzing mannan which may have a DP of 10 to 40 or higher to oligosaccharides having a DP of 2 to 10.

Enzyme hydrolysis may be conducted by suspending mannan material in an aqueous medium and adding appropriate commercially available enzymes, such as for example, cellulase and hemicellulase. Enzyme hydrolysis may be carried out using standard conditions as known by one of ordinary skill in the art.

Microbial fermentation may also be used to hydrolyze mannan material. Mannan material may be fermented with microorganisms that produce enzymes capable of hydrolyzing mannan. Microorganisms which produce enzymes such as cellulase and hemicellulase may be used. One example of an appropriate microorganism is Basidiomycota.

Resulting hydrolysates of mannan material may be used as is or be further purified with activated carbon, adsorptive resins, ion-exchange resin, ion-exchange membranes, and combinations thereof. Decoloration and/or deodorization of hydrolysates may be accomplished by contacting the hydrolysate with activated carbon or any other similar type of adsorptive resin material. Desalting and deacidification may be conducted using ion-exchange resins and/or ion-exchange membranes. Combinations of these methods may also be used. Further purification may be conducted at higher dosage levels or when the hydrolysate is to be used in certain type of foods or beverages.

### Clinical Tests

In testing conducted with MOS preparations, body weight and waist circumference tended to decrease in the MOS groups at 12 weeks compared to start time. On average, body weight loss ranged from 0.5 to 1.0 kg, and waist circumference declined by 1.5 to 3.0 cm. Body fat ratios by DEXA decreased between 4% and 8% in the MOS groups at 12 weeks compared to start time and were significantly different from placebo group at the end of the observation period. Abdominal total fat area and visceral fat area by CT-scan were reduced significantly in the MOS groups compared to the placebo group at 12 weeks, with decreases in fat measures averaging 10% to 15% from the start of the study. Characterization of the CT-scan fat measures on a relative basis further demonstrated the significant changes in the abdominal region. The results showed that coffee beverage containing MOS reduced body fat ratios and abdominal fat in humans that, over time, may be reflected in reduced body weight.

While not intending to be bound by any theory, a reduction in abdominal fat may be due to one or more of the following reasons: (1) propionic acid, which is an intestinal fermentation product of MOS, may suppress fatty acid synthesis in the liver, (2) MOS may inhibit the absorption of lipids in the small intestine causing fat accumulated in the body to be metabolized and (3) fat excretion through fecal volume may increase thus reducing abdominal fat.

### EXAMPLES

### EXAMPLE 1: MOS Clinical Trials - Study 1

### 1. MOS Preparation

Spent coffee grounds are hydrolyzed in a reactor kept at a temperature of 220°C. The hydrolyzed product is decolorized by activated carbon powder (Umehachi; Taihei Chemicals, Osaka), and desalted by cation exchange resin (SKIB; Mitsubishi Chemicals, Tokyo) and anion exchange resin (WA30; Mitsubishi Chemicals). Monosaccharides are eliminated using active carbon chromatography with a stepwise gradient of water and 10.0% (v/v) ethanol. The purified solution is concentrated in a rotary evaporator and lyophilized. The MOS mixture (degree of polymerization (dp) 2:21.2%, dp3:22.2%, dp4:21.2%, dp5:16.2% and dp6 or more: 19.2%) is used for clinical test.

### 2. Test Beverages

**The compositions of coffee beverages is as follows.**

**Table 1**

| **g/100ml** | **Control Coffee** | **MOS Coffee** |
|---|---|---|
| coffee solids | 1.1 | 1.1 |
| artificial sweetener | 0.017 | 0.017 |
| MOS | -- | 1.0 |
| corn syrup solids | 1.0 | -- |

The coffee beverage is packed in 900 ml PET (polyethylene terephthalate) bottle. The test beverage contains 1 g of MOS in 100 ml. Each subject ingested 300 ml of the test coffee beverage (containing MOS 3.0 g) per day. Corn syrup solid is added to the control coffee beverage as a placebo instead of MOS. The test coffee beverages are prepared to be same coffee solid level so that there is no difference in flavor between them. Both beverages are also prepared to equalize caffeine levels because caffeine is known to increase energy expenditure. Caffeine level is 45.4 mg/100 ml in the control coffee and 46.8 mg/100 ml in the test coffee, respectively.

### 3. Subject and Test Schedule.

This study is designed as a placebo-controlled, double-blind test. Volunteers undergo a medical and a physical examination. A fasting blood sample is collected for serum lipid analysis.

Thirty volunteers (male: 15, female: 15) are selected. They belong to obese category 1 (25 kg/m² < BMI < 30 kg/m²) according to the classification of obese by Japan Society for the Study of Obesity and hypercholesterolemic category (serum total cholesterol level > 180 mg/100 ml). They are divided into two groups considering BMI and serum total cholesterol level by a doctor not related to this study.

After a one-week observation period, subjects are administered a coffee beverages for 12 weeks. They visit the clinic at the start day, after 4, 8 and 12 weeks. On each visit to the clinic, they undergo a medical and physical examination. Fasting blood and urine samples are collected and abdominal fat areas are measured using computed tomographic scanning (CT-scan).

The subjects measure 300 ml of coffee beverage (MOS or Placebo) by measuring cup and drink it every day without adding milk or cream to it. The intake time is not mandated. The subjects are instructed that throughout the study they are not to change their ordinary diets in any way. In addition, they are restricted from consuming foods and medicines that would influence blood lipid levels. On each visit to the clinic, they are required not to eat and drink except drinking water from AM 9:00 until they complete the examinations.

### 4. Dietary Data Recording

A weight scale, a disposable camera and a pedometer are provided to the subjects. The subjects are required to record all meals, including between-meal snacks, the weight of each dish, take photos of each meal and count the number of steps walked each day for 3 days prior to visiting the clinic. The nutrient intakes were calculated from the meal records and photos using a Microsoft Excel Add-in "Excel Eiyou-kun ver.3.0" (Kenpakusya; Tokyo, Japan). The volunteers also record their alcohol intake on a daily basis.

### 5. Physical Examinations

Physical examinations are conducted to record body height, weight, circumferences of hip and waist and thickness of subcutaneous fat. Circumference of hip and waist were measured at exhalation and standing position according to the method of Japan Society for the Study of Obesity (Tokunaga et al., Int. J. Obesity, 7, 437-445(1983)). Thickness of subcutaneous fat was measured at brachial and subscapular using a caliper. Body Mass Index (BMI) was calculated according to the following equation: BMI (Kg/m²)= [Body weight (Kg)] / [Body height (m)]².

### 6. Abdominal Fat Area Measurement

Determinations of abdominal fat areas by CT-scan is carried out using the Tokunaga method (Int. J. Obesity, 7, 437-445(1983)). All CT scans are made using a CT-W450 (Hitachi Medico Inc., Japan). X-ray photographs are shot using tube voltage: 120 kV, tube current: 90 mA; window level: 0; and window width: 1000. Total fat areas, subcutaneous fat areas and visceral fat areas are determined from the CT photos using a visceral fat instrument PC soft "Fat Scan ver. 2" (N2 System Inc., Japan).

### 7. Blood Analysis

Blood analysis in this study included white blood cell, red blood cell, hemoglobin, MCV, MCH, MCHC and platelets. Total protein, A/G ratio, album, total bilirubin, AST (GOT), ALT (GPT), ALP, LFH, γ-GTP, total cholesterol, HDL-cholesterol, LDL-cholesterol, triglyceride, uric acid, blood urea nitrogen, creatinine, Na, K, Cl, Ca, IP, Mg, famished blood sugar, total lipids, nonesterified fatty acids, lipid peroxide, HbA_{1c} and insulin are measured suing blood biochemical test.

### 8. Statistical Analysis

The results of the analysis are presented as mean values with standard errors. Comparisons of the mean values between each group are analyzed using paired t-test when difference of the data conform to normal distribution and analyzed using Wilcoxon signed-ranks test when difference of the data did not conformed to normal distribution. Variance of fat area during test period are presented as relative value compared to the data at 0 week. Differences in the mean values between two groups are subjected to an analysis of variance, and then were calculated using either student's t-test or Welch's t-test. P values < 0.05 were considered significant.

### 9. Diet During the Test Period

Statistical analysis is conducted on 29 subjects who are divided into two groups; one group of 15 subjects that was administered the control beverage (hereinafter called the "control group"); and another group of 14 subjects that was administered the beverage which included MOS (hereinafter called the "test group"). One subject was excluded from the test group due to his extraordinarily irregular diet pattern during the test period. The administration record shows that the intake rate was 99.9% for the control group and 99.7% for the test group, indicating no significant difference between the two groups. No significant intake of alcohol or medication was acknowledged during the test period. The results of the diet investigation revealed that there was little significant difference in diet during the test period for both groups. The average energy intake per day ± the standard error was 1997 ± 77 kcal/day for the control group and 2066 ± 72 kcal/day for the test group. The level of exercise, as indicated by the pedometers, indicated no significant change in either group during the test period.

### 10. Side Effects

No side effects related to the administration of the test beverages were observed in the physical condition of the subjects at any time during the test period. Consultation by doctors identified no subjective/objective symptoms or abnormal observations due to the administration of the beverages during the test period.

### 11. Physical Measurements

**Table 2 Characteristics of subjects before test beverage ingestion**

| | | **Group** | **Total** | **Male** | **Female** |
|---|---|---|---|---|---|
| **No. of subject** | | Control | 15 | 7 | 8 |
| | | MOS | 14 | 7 | 7 |
| | | | | | |
| Age | y | Control | 55.7±2.8 | 53. 1±14.1 | 57.9±7.2 |
| | | MOS | 49.6±2.9 | 46.0±11.5 | 53.1±9.8 |
| | | | | | |
| Height | cm | Control | 157.2±2.1 | 163.2±5.8 | 151.9±5.7 |
| | | MOS | 160.3±2.3 | 167.5±1.8 | 153.2±5.8 |
| | | | | | |
| Weight | kg | Control | 66.9±1.8 | 71.8±6.3 | 62.5±4.8 |
| | | MOS | 69.2±2.0 | 74.5±3.9 | 63.9±6.5 |
| | | | | | |
| BMI | kg/m² | 2 Control | 27.0±0.2 | 26.9±0.8 | 27.1±1.0 |
| | | MOS | 26.9±0.4 | 26.6±1.4 | 27.1±1.4 |
| | | | | | |
| Body Temp | °C | Control | 36.2±0.1 | 36.3±0.3 | 36.1±0.5 |
| | | MOS | 36.0±0.1 | 36.1±0.4 | 35.8±0.4 |
| | | | | | |
| Waist | cm | Control | 86.3±1.2 | 89.1±2.6 | 83.8±4.7 |
| | | MOS | 86.5±1.5 | 88.6±4.2 | 84.4±6.1 |
| | | | | | |
| Hip | cm | Control | 96.8±1.0 | 96.1±3.3 | 97.4±4.2 |
| | | MOS | 97.8±0.9 | 97.3±2.3 | 98.2±4.1 |
| Thickness of subcutaneous | cm | Control | 1.8±0.1 | 1.4±0.4 | 2.1±0.3 |
| fat at brachial | | MOS | 1.7±0.1 | 1.4±0.1 | 2.0±0.2 |
| | | | | | |
| Thickness of subcutaneous | cm | Control | 2.3±0.2 | 2.7±1.1 | 2.0±0.3 |
| fat at subscapular | | MOS | 2.3±0.1 | 2.3±0.3 | 2.3±0.3 |
| | | | | | |
| SBP^{a} | mmHg | Control | 127.5±4.4 | 128.1±15.6 | 127.0±19.4 |
| | | MOS | 131.8±3.3 | 132.9±11.8 | 130.7±13.6 |
| | | | | | |
| SBP^{b} | mmHg | Control | 79.9±2.3 | 81.6±9.7 | 78.4±8.6 |
| | | MOS | 82.4±2.3 | 84.3±8.6 | 80.4±9.9 |
| | | | | | |
| Pulse | beat/min | Control | 69.3±2.2 | 67.1±6.9 | 71.3±9.8 |
| | | MOS | 67.8±1.6 | 67.7±6.6 | 67.9±5.6 |

| | | | | | |
|---|---|---|---|---|---|
| Values presented as means and standard errors. ^{a}Systolic blood pressure ^{b}Diastolic blood pressure | | | | | |

No significant difference was acknowledged between the control group and the test group. The changes in physical measurements during the test period are indicated below.

**Table 3 Change of physical examination items during administration period**

| | | **0 week** | **4 weeks** | **8 weeks** | **12 weeks** |
|---|---|---|---|---|---|
| Weight (kg) | Control | 66.9 ± 1.8 | 66.3 ± 1.8 | 66.5 ± 1.9 | 66.3 ± 1.9 |
| | MOS | 69.2 ± 2.0 | 68.8 ± 1.9 | 68.6 ± 2.0 | 68.7 ± 2.0 |
| BMI (kg/m²) | Control | 27.0 ± 0.2 | 26.8 ± 0.3 | 26.9 ± 0.3 | 26.8 ± 0.3 |
| | MOS | 26.9 ± 0.4 | 26.7 ± 0.3 | 26.6 ± 0.4 | 26.7 ± 0.3 |
| Waist (cm) | Control | 86.3 ± 1.2 | 85.6 ± 1.3* | 85.1 ± 1.2** | 85.1 ± 1.4* |
| | MOS | 86.5 ± 1.5 | 86.0 ± 1.4 | 85.1 ± 1.6 | 84.8 ± 1.5* |
| Hip (cm) | Control | 96.8 ± 1.0 | 96.4 ± 1.0* | 96.5 ± 1.0 | 96.2 ± 1.1 |
| | MOS | 97.8 ± 0.9 | 97.6 ± 0.9 | 96.4 ± 1.2 | 96.1 ± 1.2 |
| Thickness of subcutaneous at brachial (cm) | Control | 1.8 ± 0.1 | 1.8 ± 0.1 | 1.8 ± 0.1 | 1.8 ± 0.1 |
| | MOS | 1.7 ± 0.1 | 1.7 ± 0.1 | 1.8 ± 0.1 | 1.7 ± 0.1 |
| Thickness of subcutaneous at subscaplur (cm) | Control | 2.3 ± 0.2 | 2.4 ± 0.2 | 2.4 ± 0.2 | 2.3 ± 0.2 |
| | MOS | 2.3 ± 0.1 | 2.3 * 0.1 | 2.3 ± 0.1 | 2.2 * 0.1. * |

| | | | | | |
|---|---|---|---|---|---|
| Values presented as means and standard erros. *,** Significantly different from before ingestion 0 week at *p* <0.05 *p* <0.01, respectively. | | | | | |

There was no significant difference in weight, BMI or subcutaneous fat in the middle part of the brachial extension side for either group. The reduction in waist size for both groups was statistically significant when compared to before the administration of the beverages, however no significant difference was identified between the two groups. The thickness of the subcutaneous fat near the dorsal subscapular for the test group was significantly reduced (p<0.05) in the 12th week compared to the start of administration, but there was no significant difference between the two groups. The differences between the two groups were acknowledged as minimal and insignificant.

### 12. Abdominal Fat Area by CT Scan

One subject from the control group and one subject from the test group are excluded from the analysis of the cross section of the lipids, as a perfect CT scan image could not be obtained due to the subjects' large waist size. Statistical analysis is conducted on 27 subjects, 14 from the control group and 13 from the test group. The change in abdominal fat area during the test period is indicated in Table 4.

**Table 4 Changes of abdominal fat areas during administration**

| | | **0 week** | **4 weeks** | **8 weeks** | **12 weeks** | ***p* value or repated measure ANOVA (Test period)** |
|---|---|---|---|---|---|---|
| Total fat area (cm²) | Control | 274.5 ± 11 | 263 ± 12.5 | 261 ± 11.6 | 265.5 ± 11.1 | |
| | Δ (From 0W) | | -11.3 ± 7.1 | -13.8 ± 6.8 | -8.9 ± 6.1 | |
| | MOS | 281.5 ± 19.1 | 266.3 ± 19.8 | 251.0 ± 21.6 ** | 248.9 ± 19.2** | 0.00003 |
| | Δ (From 0W) | | -15.2 ± 7.7 | -30.5 ± 4.4 | -32.6 ± 5.4 †† | |
| Subcutaneous fat area (cm²) | Control | 181.6 ± 12.3 | 169.8 ± 13.0 | 171.4 ± 12.S * | 174.6 ± 13.1 | |
| | Δ (From 0W) | ± | -11.7 ± 5.7 | -10.1 ± 4.3 | -7.0 ± 6.0 | |
| | MOS | 173.4 ± 15.1 | 166.1 ± 15.1 | 156.7 ± 15.7** | 154.5 ± 14.8** | 0.00024 |
| | Δ (From 0W) | ± | -7.3 ± 4.4 | -16.8 ± 3.7 | -18.9 ± 3.3 | |
| Visceral fat ares (cm²) | Control | 92.9 ± 8.4 | 93.1 ± 6.6 | 89.2 ± 8.3 | 91.0 ± 9.7 | |
| | Δ (From 0W) | ± | 0.4 ± 4.4 | -3.7 ± 4 | -9.0 ± 6.1 | |
| | MOS | 108 ± 11 | 100 ± 10.8 | 94.4 ± 11.3** " | 94.4 ± 11.9** | 0.01928 |
| | Δ (From 0W) | ± | -7.9 ± 5.2 | -13.7 ± 2.9 | -13.7 ± 3.9 t | |

| | | | | | | |
|---|---|---|---|---|---|---|
| Values presented as means and standard erros. *,** Significantly different from before ingestion 0 week at *p* <0.05, p <0.01, respectively. †,†† Significantly different from the control group at p<0.05, p<0.01, respectively. | | | | | | |

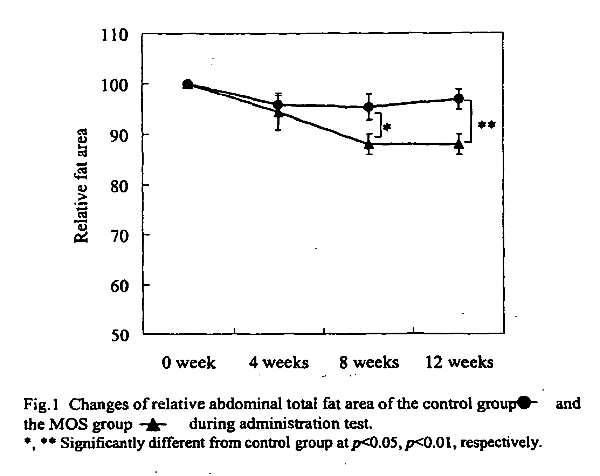

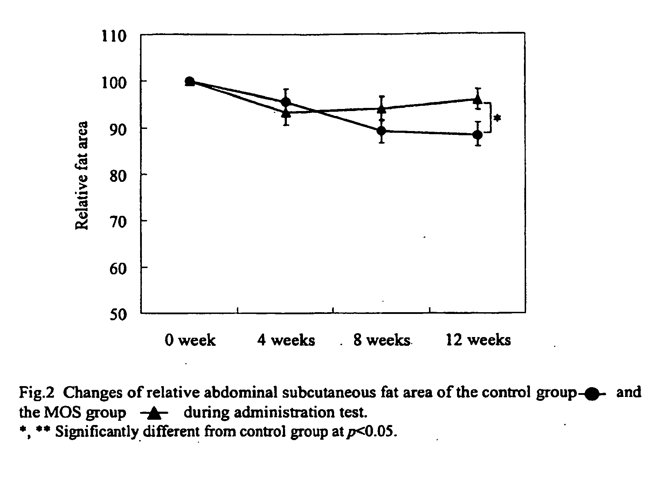

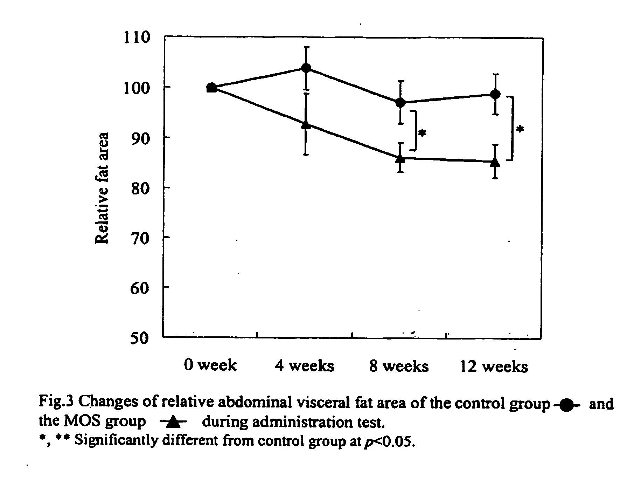

No significant difference was identified between the groups in terms of total fat area, subcutaneous fat area and visceral fat area at the start of administration (week 0). During the test period of the fat area analysis, the subcutaneous fat area of the control group was significantly reduced in the 8th week (p<0.05) compared to the start of administration. In the test group, a significant reduction (p<0.01) was identified in the 8th and 12th weeks regarding total fat area, subcutaneous fat area and visceral fat area. The diminution from before ingestion 0 week in the MOS group was larger than that in the control group. The test group clearly showed significantly lower values for total fat area in the 8th week (p<0.05) and 12th week (p<0.01) compared to the control group (Fig.1). The test group also showed a significant reduction in the subcutaneous fat area and visceral fat area in the 12th week (p<0.05 for both) compared to the control group. (Fig. 2 & 3)

### 13. Results of the Blood Test

Two subjects from the test group are excluded from the blood analysis, as they had breakfast and soft drinks from AM 9:00 on the 12^{th} week visit to the clinic. The changes in the values of the blood examination during the test period are indicated in Table 5, and the changes in the values of the blood biochemical examination are indicated in Table 6.

**Table 5 Results of blood tests during administration period**

| | **Normal range** | **unit** | **Group** | **0 week** | **4 weeks** | **8 weeks** | **12^{.}weeks** |
|---|---|---|---|---|---|---|---|
| White blood cell | 3500-9700 | /µL | Control | 6553 ± 1015 | 6544 ± 1156 | 6313 ± 1483 | 6303 ± 1279 |
| | | | MOS | 6748 ± 1721 | 6490 ± 1608 | 6415 ± 1658 | 6872 ± 1279 |
| | | | | | | | |
| Red blood cell | M438 - 577 | ×10⁴/µL | Control | 464.9 ± 40.5 | 468.5 ± 42.6 | 457.1 ± 40.4* | 460.9 ± 38.6 |
| | F376- 516 | | MOS | 486.6 ± 32.3 | 487.4 ± 31.1 | 473.1 ± 34.1* | 475.3 ± 38.6** |
| | | | | | | | |
| Hemglobin | M13.6-18.3 | g/100ml | Control | 14.17 ± 1.52 | 14.29 ± 1.63 | 14.00 ± 1.48 | 14.12 ± 1.43 |
| | FI1.2-15.2 | | MOS | 14.92 ± 0.95 | 14.82 ± 0.90 | 14.70 ± 0.96* | 14.38 ± 1.45** |
| | | | | | | | |
| Hematocrit | M40.4-51.9 | % | Control | 44.29 ± 4.39 | 44.61 ± 4.69 | 43.31 ± 4.60** | 43.59 ± 4.15* |
| | F34.3-45.2 | | MOS | 45.94 ± 2.92 | 46.31 ± 2.55 | 45.19 ± 2.68 | 44.81 ± 4.15* |
| | | | | | | | |
| MCV | M83 - 101 | fL | Control | 95.3 ± 3.7 | 95.2 ± 3.0 | 94.7 ± 3.9 | 94.6 ± 3.3 |
| | F80 - 101 | | MOS | 94.4 ± 3.5 | 95.0 ± 3.4 | 95.2 ± 2.6 | 94.4 ± 3.3 |
| | | | | | | | |
| MCH | M28.2 - 34.7 | P8 | Control | 30.47 ± 1.37 | 30.46 ± 1.31 | 30.61 ± 1.28 | 30.62 ± 1.19 |
| | F26.4-34.3 | | MOS | 30.69 ± 0.84 | 30.45 ± 0.96 | 30.95 ± 0.74 | 30.67 ± 1.19 |
| | | | | | | | |
| MCHC | M31.8-36.4 | % | Control | 32.01 ± 1.47 | 32.02 ± 0.71 | 32.35 ± 0.51 | 32.38 ± 0.79 |
| | F31.3-36.1 | | MOS | 32.50 ± 0.91 | 32.01 ± 1.01 | 32.53 ± 0.59 | 32.34 ± 0.79 |
| | | | | | | | |
| Platelet | 14.0.37.9 | ×10⁴/µL | Control | 24.32 ± 4.43 | 25.09 ± 5.41 | 23.32 ± 3.99 | 24.33 ± 4.87 |
| | | | MOS | 23.02 ± 4.85 | 23.85 ± 4.54 | 22.58 ± 4.82 | 23.36 ± 4.87 |

| | | | | | | | |
|---|---|---|---|---|---|---|---|
| Values presented as means and standard erros. *,** Significantly different from before ingestion 0 week at *p* <0.05.*p* <0.01, respectively. | | | | | | | |

**Table 6 Results of blood biochemical test during administration period**

| | Normal range | Unit | Group | 0 week | 4 weeks | 8 weeks | 12 weeks |
|---|---|---|---|---|---|---|---|
| Total protein | 6.5 - 8.2 | g/100 ml | Control | 7.48 ± 0.09 | 7.46 ± 0.33 | 7.33 ± 0.33** | 7.34 ± 0.28** |
| | | | MOS | 7.53 ± 0.09 | 7.51 ± 0.19 | 7.53 ± 0.26 | 7.40 ± 0.28 |
| | | | | | | | |
| A/G ratio | 1.30 - 2.00 | - | Control | 1.45 ± 0.19 | 1.43 ± 0.16 | 1.45 ± 0.16 | 1.49 ± 0.16 |
| | | | MOS | 1.51 ± 0.18 | 1.44 ± 0.15 | 1.45 ± 0.17 | 1.48 ± 0.16 |
| | | | | | | | |
| Albumin | 3.7 - 5.5 | g/100 ml | Control | 4.41 ± 0.24 | 4.38 ± 0.24 | 4.33 ± 0.22* | 4.37 ± 0.14 |
| | | | MOS | 4.50 ± 0.19 | 4.42 ± 0.24 | 4.44 ± 0.29 | 4.41 ± 0.14* |
| | | | | | | | |
| Total bilirubin | 0.2 - 1.0 | mg/100 ml | Control | 0.79 ± 0.22 | 0.71 ± 0.21 | 0.74 ± 0.24 | 0.79 ± 0.25 |
| | | | MOS | 0.72 ± 0.20 | 0.67 ± 0.18 | 0.74 ± 0.22 | 0.81 ± 0.25 |
| | | | | | | | |
| AST (GOT) | 10-40 | U/l | Control | 25.9 ± 9.9 | 25.0 ± 9.2 | 25.0 ± 8.2 | 24.6 ± 7.0 |
| | | | MOS | 22.8 ± 101 | 20.4 ± 7.0 | 22.5 ± 7.6 | 23.3 ± 7.0 |
| | | | | | | | |
| ALT (GPT) | 5-45 | U/l | Control | 28.1 ± 16.7 | 28.1 ± 19.0 | 25.5 ± 11.4 | 28.1 ± 11.7 |
| | | | MOS | 28.3 ± 17.8 | 25.2 ± 9.7 | 25.8 ± 13.2 | 28.0 ± 11.7 |
| | | | | | | | |
| ALP | 104 - 338 | U/l | Control | 238.7 ± 64.4 | 245.9 ± 73.6 | 228.4 ± 65.3 | 228.5 ± 64.2 |
| | | | MOS | 214.3 ± 55.5 | 223.3 ± 52.8 | 209.2 ± 52.6 | 208.1 ± 64.2 |
| | | | | | | | |
| LDH | 120-245 | U/l | Control | 205.5 ± 25.5 | 210.3 ± 29.0 | 215.1 ± 25.4** | 202.2 ± 23.8 |
| | | | MOS | 206.3 ± 50.7 | 201.6 ± 41.2 | 210.6 ± 43.6 | 196.6 ± 23.8* |
| | | | | | | | |
| y -GTP | M16-73 | U/l | Control | 37.2 ± 27.1 | 39.9 ± 28.9 | 35.6 ± 23.2 | 41.9 ± 30.6 |
| | (F8-32) | | MOS | 38.7 ± 21.7 | 36.2 ± 20.2 | 32.1 ± 15.1* | 39.0 ± 30.6 |
| | | | | | | | |
| T-cholesterol | 150-219 | mg/100ml | Control | 236.9 ± 28.2 | 235.3 ± 22.8 | 233.9 ± 30.2 | 243.5 ± 26.6 |
| | | | MOS | 223.2 ± 30.3 | 233.3 ± 32.7 | 219.4 ± 30.6 | 224.5 ± 26.6 |
| | | | | | | | |
| HDL-Cho | M40-80 | mg/100 ml | Control | 52.8 ± 11.7 | 52.5 ± 13.2 | 52.5 ± 12.7 | 51.3 ± 13.7 |
| | F40-90 | | MOS | 56.6 ± 13.3 | 55.0 ± 17.6 | 56.1 ± 13.1 | 54.7 ± 13.7 |
| | | | | | | | |
| LDL-Cho | 70-139 | mg/100ml | Control | 163.1 ± 30.3 | 151.3 ± 26.5 | 157.4 ± 28.5 | 156.8 ± 23.0 |
| | | | MOS | 151.3 ± 26.0 | 152.2 ± 23.8 | 146.4 ± 28.7 | 141.8 ± 23.0* |
| | | | | | | | |
| Triglyceride | 50-149 | mg/100ml | Control | 127.5 ± 56.6 | 153.0 ± 100.7 | 146.8 ± 70.0 | 142.3 ± 84.9 |
| | | | MOS | 105.9 ± 52.5 | 139.0 ± 77.5 | 119.3 ± 52.2 | 113.2 ± 84.9 |

At the start of administration (week 0), no significant difference was identified between the two groups in any of the items of the blood examination and blood biochemical examination. For inspection items related to human serum lipids, the results of the blood examination and blood biochemical examination of the test group show a significant drop in LDL-cholesterol between the start of administration and the 12th week (p<0.05). Although no significant difference was identified between the two groups, the test group had a tendency to show lower values for total cholesterol concentration (p<0.054) and LDL-cholesterol (p <0.062) in the 12th week, compared to the control group. Although compared to the start of administration there were several statistically significant variations in items other than those related to human serum lipids, such variations were within the standard range.

### EXAMPLE 2: Effect of Liquid Coffee Containing Mannooligosaccharides

Data from test subjects in Example 1 who had a BMI>26.4 (2 male and 5 female) is further analyzed. After drinking liquid coffee containing MOS, body weight, body fat ratio and BMI dropped significantly as shown in the table. Liquid coffee containing MOS (3 g/300ml) is thus shown to have the body fat reduction or anti-obesity effects in humans.

**Table Effects of liquid coffee containing MOS intake on body weight, body fat ratio and BMI**

| Intake period | 0 week | 4 weeks | 8 weeks | 12 weeks |
|---|---|---|---|---|
| Body Weight (kg) | 68.8±8.1 | 68.0±7.5 | 67.9±2.2* | 67.8±2.2* |
| Body Fat Ratio (%) | 35.4±4.5 | 33.7±4.5** | 32.3±1.3* | 33.8±1.1* |
| BMI | 27.8±0.7 | 27.5±0.8 | 27.5±0.3* | 27.4±0.3° |

| | | | | |
|---|---|---|---|---|
| ** significantly different from before intake (p<0.01) * significantly different from before intake (p<0.05) □significantly different from before intake (p<0.10) | | | | |

### EXAMPLE 3: MOS Clinical Trials - Study 2

This study is similar to the study described in Example 1 except that two doses of MOS are used (MOS 3.0 or 6.0 grams/day). All measurements are the same as described in Example 1 with the addition of dual energy X-ray absorptiometry (DEXA).

This study is designed as placebo-controlled, double-blind test. Volunteers undergo a medical and a physical examination. A fasting blood sample is collected for serum lipid analysis prior to the starting the study.

Seventy-two volunteers (36 males; 36 females) are selected as subjects for the test. Volunteers are categorized as obese category 1 (25 kg/m² < BMI < 30 kg/m²) according to the classification of obesity by Japan Society for the Study of Obesity. They are divided into three groups considering BMI and initial body fat ratios by a doctor not related to this study.

After a one-week observation period, subjects are administered the coffee beverage for 12 weeks. The subjects measure 300 ml of coffee beverage (MOS or Placebo) by measuring cup and drink it every day without adding milk or cream. The beverage is taken with a meal. The subjects are instructed that throughout the study they are not to change their ordinary diets in any way. In addition, they are restricted from consuming foods and medicines that would influence blood lipid levels.

Subjects visit the clinic at day 0, and then after 4, 8 and 12 weeks. On each visit to the clinic, they are required not to eat and drink except drinking water from AM 9 until examinations are complete. On each visit, subjects undergo a medical and physical examination. Total body fat ratios are measured by dual energy X-ray absorptiometry (DEXA). In addition, at day 0 and 12 weeks, fasting blood and urine samples are collected and abdominal fat areas are measured using computed tomographic scanning (CT-scan).

Results of the present Study 2 are similar in part to results in Study 1 (Example 1). Losses in total body weight, waist circumference, body fat ratios, abdominal fat and visceral fat all occur in the MOS group. Further, total body fat determined by DEXA decreases an average of 6% in the MOS group at 12 weeks as compared to the start of the study, and was significantly different than the control group. No dosage related responses are noted based on experimental work to date.

### EXAMPLE 4: MOS Effect on Pancreatic Lipase Activity

MOS compositions were prepared as indicated in Example 1. The DP distribution of mannooligosaccharides (MOS) composition used for this example was DP1: 2.4%; DP2: 26.6%; DP 3: 20.2%; DP 4: 17.8%; DP 5: 10.9%; DP 6: 8.9%; DP 7: 6.0%; DP8: 3.6%; DP 9: 1.9%; and DP 10: 1.7%. The content of mannose residues in the sugar chain was 90%.

### Measurement of pancreatic lipase activity

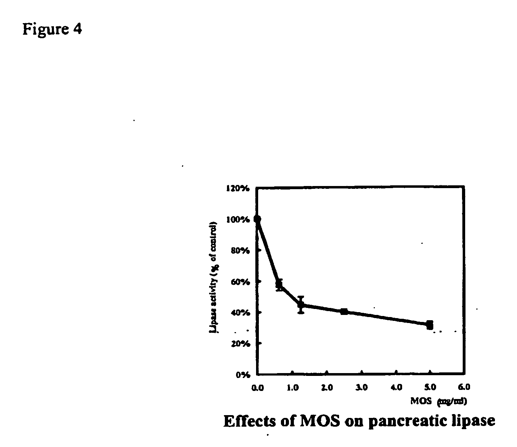

Lipase activity was determined by measuring the rate of release of oleic acid from triolein. Briefly, a suspension of triolein (80mg), phosphatidylcholine (10mg) and taurocholic acid (5mg) in 9 ml of 0.1M *N*-tris(hydroxymethyl)methyl-2-aminoethanesulfonic acid (TES) buffer (pH 7.0) containing 0.1 m NaCl was sonicated for 5 minutes. This sonicated substrate suspension (100µl) was incubated with 50µl (10 units) of pancreatic lipase and 100µl of various concentrations of MOS solution for 30 minutes at 37°C in a final volume 250µl. The amount of released oleic acid was determined by the normal method. The incubation mixture was added to 3 ml aliquots of a 1:1 (v/v) mixture of chloroform and n-heptane containing 2% (v/v) methanol and extracted by shaking the tubes horizontally for 10 minutes, and the upper aqueous phase removed by suction. Copper reagent (1 ml) was then added to the lower organic phase. The tube was shaken for 10 min, the mixture was centrifuged at 2000g for 10 minutes, and 0.5ml of the upper organic phase, which contained copper salts of the extracted free fatty acids, was treated with 0.5ml of 0.1% (w/v) bathocuproine in chloroform containing 0.05% (w/v) 3(2)-t-butyl-4-hydroxyanisole. The absorbance was then measured at 480nm. Lipase activity was expressed per ml of reaction per hour. As shown in Figure 4, MOS inhibited the pancreatic lipase activity dose-dependently at concentrations of 1-5mg/ml; at 5 mg/ml, it inhibited about 70%. These *in vitro* results suggest that MOS is strong agent that inhibits lipid absorption.

### EXAMPLE 5: Plasma Triglyceride After Intake of High Fat Diet in Human Study

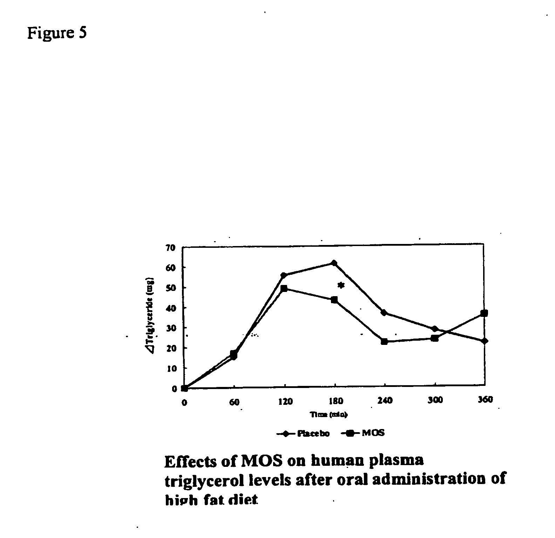

Ten healthy human volunteers (all males) are divided into 2 groups and a double-blind cross-over design is adopted. Each group is administered MOS or placebo in the first set. After 1 week, second set (placebo or MOS) is administered vice versa. They are fasted overnight and orally administered 200g of high-fat diet consisting of com potage soup containing butter and lard (40g of fat content) with or without MOS (3g). Blood is collected from vein before and after (1h, 2h, 3h, 4h, 5h and 6h) the oral administration and subjected to measurement of triglyceride. As shown in Figure 5, the plasma triglycerides increase was significantly suppressed after 3 hours administration. MOS intake in high fat diet was effective to reduce the postprandial plasma triglyceride increase. These results suggest that MOS intake is effective for reducing the fat uptake in the human body.

### EXAMPLE 6: Effect of Mannooligosaccharides on Body Fat Ratios

The effect of MOS on human body fat ratio was investigated using healthy Japanese subjects (6 male and 7 female). One group consisting of 6 subjects took MOS at 1.0 g/day. A second group consisting of seven subjects took MOS at 3.0 g/day. Evaluation was made based on change in body fat ratio. Body fat ratio was analyzed using electric impedance methodology. The table below shows the results of the effect of MOS intake on body fat ratio.

After taking MOS, body fat ratio dropped as shown in the table below. MOS (1.0 g/day and 3.0g/day) is thus shown to have the body fat reduction effect in humans.

| Change of Body Fat Ratio (%) | | |
|---|---|---|
| MOS 1.0g/day | | MOS 3.0g/day |
| N | 6 | 7 |
| Initial | 31.1±5.9 | 31.4±4.3 |
| 2 weeks | 30.7±4.4 | 31.2±4.7 |
| 4 weeks | 30.1±5.1 | 30.3±4.7* |

| | | |
|---|---|---|
| *significantly different from before intake (p,0.05) | | |

### EXAMPLE 7: Effect of Liquid Coffee Containing Mannooligosaccharides

The effect of liquid coffee containing MOS on human body weight and body fat ratio was investigated using ten healthy Japanese subjects using the same coffee samples containing MOS as described in Example 6. Subjects consumed the liquid coffee containing MOS and drank 900 ml of liquid coffee every day for 4 weeks (i.e., 3 bottles/day for a total of 9g of MOS/day). Evaluation was made based on change in body weight, body fat ratio and BMI as in Example 6. The table below shows the results of the effect of liquid coffee containing MOS intake on body weight, body fat ratio and BMI.

After drinking liquid coffee containing MOS, body weight, body fat ratio and BMI dropped significantly as shown in Table. Liquid coffee containing MOS is thus shown to have the body fat reduction or anti-obesity effect in humans.

Effects of Liquid Coffee Containing MOS on Body Wieght, Body Fat Ratio and BMI

| | | 0w | 2w | 4w |
|---|---|---|---|---|
| Body Weight | kg | 59.5 ± 12.2 | 58.9 ± 12.0* | 59.1 ± 12.2* |
| Body fat ratio | % | 24.2 ± 4.3 | 23.8 ± 4.8 | 23.4 ± 4.6* |
| BMI | | 24.4 ± 2.9 | 22.2 ± 2.9* | 22.2 ± 3.0* |

| | | | | |
|---|---|---|---|---|
| n=10, *significantly different from before intake (p<0.05) | | | | |

## Claims

1. A composition comprising a mannooligosaccharide having a degree of polymerisation of 2 to 10 monosaccharides, wherein at least 60 weight percent of the monosaccharides are mannose, for use in reducing body fat ratios or abdominal fat in a human.

2. A mannooligosaccharide composition for use according to Claim 1, wherein the mannoooligosaccharide composition is orally administered at a rate of 1 to 10 grams per day.

3. A mannooligosaccharide composition for use according to Claim 1 or 2, further comprising at least one monosaccharide selected from the group consisting of glucose, galactose, fructose, and combinations thereof.

4. A mannooligosaccharide composition for use according to any of Claims 1 to 3, wherein the mannooligosaccharide composition is a hydrolysate provided by hydrolysis of a mannan material.

## Patentansprüche

1. Zusammensetzung, die ein Mannooligosaccharid umfasst, das einen Polymerisationsgrad von 2 bis 10 Monosacchariden aufweist, worin mindestens 60 Gewichtsprozent der Monosaccharide Mannose sind, zur Verwendung bei der Reduzierung von Körperfettanteilen oder Bauchfett bei einem Menschen.

2. Mannooligosaccharidzusammensetzung zur Verwendung nach Anspruch 1, worin die Mannooligosaccharidzusammensetzung oral bei einer Rate von 1 bis 10 Gramm pro Tag verabreicht wird.

3. Mannooligosaccharidzusammensetzung zur Verwendung nach Anspruch 1 oder 2, die weiter mindestens ein Monosaccharid umfasst, das aus der Gruppe ausgewählt ist, die aus Glucose, Galactose, Fructose und Kombinationen davon besteht.

4. Mannooligosaccharidzusammensetzung zur Verwendung nach einem der Ansprüche 1 bis 3, worin die Mannooligosaccharidzusammensetzung ein Hydrolysat ist, das durch Hydrolyse eines Mannan-Stoffs bereitgestellt wird.

## Revendications

1. Composition comprenant un manno-oligosaccharide dont le degré de polymérisation est compris dans la plage de 2 - 10 monosaccharides, le mannose représentant au moins 60 pour cent en poids des monosaccharides, en vue d'une utilisation pour réduire les proportions de graisses corporelles ou les graisses abdominales chez un humain.

2. Composition comprenant un manno-oligosaccharide en vue d'une utilisation selon la revendication 1, où la composition comprenant un manno-oligosaccharide est administrée par voie orale à un taux compris dans la plage de 1 - 10 grammes par jour.

3. Composition comprenant un manno-oligosaccharide en vue d'une utilisation selon la revendication 1 ou 2, qui comprend également au moins un monosaccharide sélectionné dans le groupe consistant en le glucose, le galactose, le fructose et des combinaisons de ceux-ci.

4. Composition comprenant un manno-oligosaccharide en vue d'une utilisation selon l'une quelconque des revendications 1 à 3, où la composition comprenant un manno-oligosaccharide est un hydrolysat obtenu par hydrolyse d'un matériau de type mannane.
